Europäisches Patentamt

European Patent Office

Office européen des brevets

(19)

(11) Publication number: **0 255 011**

**A2**

(12)

# EUROPEAN PATENT APPLICATION

(21) Application number: **87110461.8**

(22) Date of filing: **20.07.87**

(51) Int. Cl.4: **C12N 15/00** , **C12N 1/20** , **A61K 37/64** , **C07K 13/00**

(30) Priority: **29.07.86 US 891469**

(43) Date of publication of application:
**03.02.88 Bulletin 88/05**

(84) Designated Contracting States:
**AT BE CH DE FR GB IT LI LU NL SE**

(71) Applicant: **MILES LABORATORIES, INC.**
**1127 Myrtle Street**
**Elkhart Indiana 46514(US)**

(72) Inventor: **Kaumeyer, John Frederick**
**5501A University Park Drive**
**Mishawaka, IN 46545(US)**
Inventor: **Kotick, Michael Paul**
**54231 Old Mill Drive**
**Elkhart, IN 46514(US)**
Inventor: **Polazzi, Joseph Odo**
**54226 Forst Grove Avenue**
**Elkhart, IN 46514(US)**

(74) Representative: **Dänner, Klaus, Dr. et al**
**c/o Bayer AG Konzernverwaltung RP**
**Patentabteilung**
**D-5090 Leverkusen 1 Bayerwerk(DE)**

(54) **Human inter-alpha-trypsin inhibitor gene.**

(57) The present invention provides cDNA sequences encoding inter-$\alpha$-trypsin inhibitor light chain and the protein produced thereby.

EP 0 255 011 A2

## HUMAN INTER-ALPHA-TRYPSIN INHIBITOR GENE

### Background of the Invention

Inter-alpha-trypsin inhibitor (ITI) is a serine protease inhibitor found in human plasma and serum. This protein has been reported to inhibit the proteolytic enzymes trypsin, chymotrypsin and neutrophil elastase. However, the specific target of the inhibitor is unknown. ITI is unique among plasma proteinase inhibitors in that it occurs as more than one molecular weight species. It is found as a high molecular weight form of 180 kilodaltons (kd) and as a lower molecular weight species of 30 kd. This lower molecular weight species has been designated HI-30. HI-30 can be released from the high molecular weight form by treatment with trypsin. The HI-30 fragment contains all of the anti-proteolytic activity of the high molecular weight form. However, it has not been demonstrated directly that the 180 kd protein is the precursor of the HI-30 protein found in plasma. It is possible that HI-30 is produced independently of the 180 kd form.

The total structure of high molecular weight ITI is unknown. However, the degradation product, HI-30, has been purified from human urine and the complete amino acid sequence determined. HI-30 is composed of 50% carbohydrate and contains two tandemly linked anti-proteolytic domains. Both domains are structurally related to the Kunitz family of low molecular weight serine protease inhibitors.

Although 180 kd ITI has been described as a single chain glycoprotein, recent experiments indicate there are multiple messenger RNAs (mRNAs) in baboon liver coding for polypeptides which compose ITI. One of these mRNAs codes for a 42 kd protein, termed the ITI light chain. This protein reacts with antibodies specific for HI-30. See Bourguinon et al, 1983. A complementary DNA (cDNA) clone of the ITI light chain has been isolated by others from a human liver mRNA library and partially sequenced to the inhibitory portion of the second domain. This work, in contrast to amino acid sequence data, showed that HI-30 is at the carboxy-terminus of the ITI light chain. See Bourguinon et al, 1985.

Using gene cloning techniques, a cDNA copy of the gene for the ITI light chain which contains the HI-30 protein coding region was isolated from a human liver mRNA library. The entire nucleotide sequence of the longest clone (identified by colony hybridization) was determined. This confirmed that the gene for HI-30 is at the 3' end of the gene, which corresponds to the carboxy terminus of the protein. The amino acid structure (which can be derived from the nucleotide sequence by translation of the genetic code) was generally in accord with data available from the previously reported HI-30 protein sequence. However, it has now been discovered that 5' to this structural gene for HI-30 is an open reading frame which encodes the serum protein, alpha-1-microglobulin. Alpha-1-microglobulin is also known as protein HC. Alpha-1-microglobulin is a 31 kd glycoprotein found in plasma, urine and cerebrospinal fluid. This protein is similar in structure to a protein isolated by other workers who designated it protein HC. For all intents and purposes, it can be considered that alpha-1-microglobulin and protein HC are derived from the same protein and the structural differences represent artifacts arising from the methods of amino acid sequence determination. Protein HC contains a yellow-brown chromophoric group and migrates as a heterogeneously charged protein upon agarose gel electrophoresis, even after removal of the sialic acid carbohydrate residues with the enzyme neuraminidase. Alpha-1-microglobulin is found in plasma both in the free form and as a complex with IgA. It has recently been suggested that alpha-1-microglobulin is important in modulating neutrophil chemotaxis. The association of HI-30 with alpha-1-microglobulin (protein HC) has not been previously reported or suggested in the literature.

### Summary of the Invention

The invention disclosed herein is directed to a cDNA sequence encoding alpha-1-microglobulin in sequence with the HI-30 coding region of the ITI light chain. Also disclosed is a fusion protein of alpha-1-microglobulin and HI-30 produced from the cloned DNA sequences.

### Brief Description of the Drawings

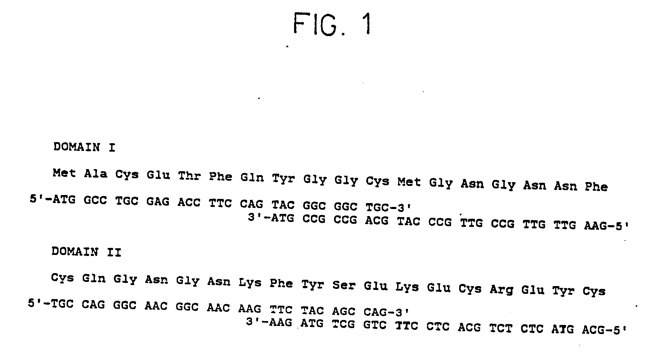

Figure 1 depicts the oligonucleotide probes used for isolation of the ITI light chain cDNA clone. The protein sequence used for designing the probes is taken from Wachter and Hachstrasser, 1981. The sequence from domain I is Met-49 to Phe-66, and that from domain II is Cys-115 to Cys-132.

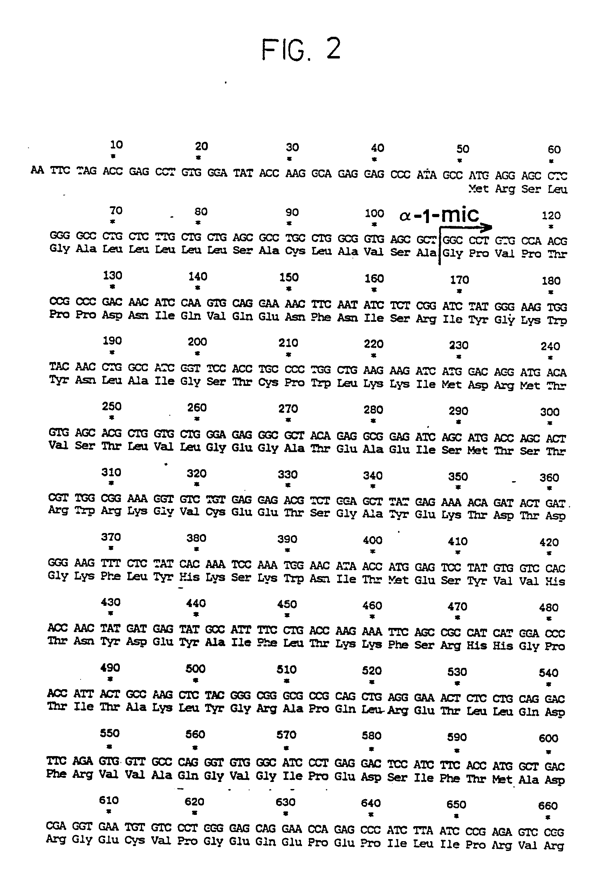

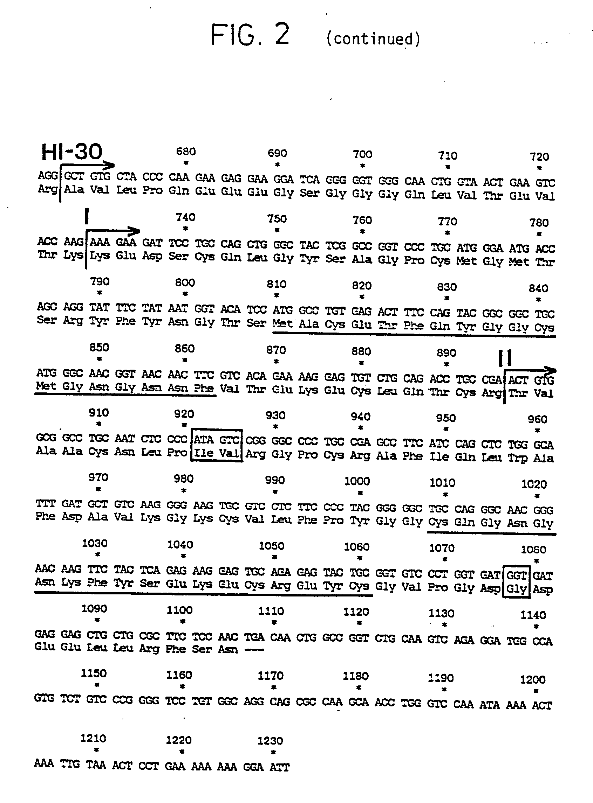

Figure 2 depicts the nucleotide and protein sequence of the ITI light chain. The sequence of α-1-microglobulin (α-1-mic) begins at nucleotide 108 and extends to nucleotide 656. HI-30 begins at nucleotide 666 and extends to nucleotide 1100. Domain I begins at nucleotide 729; domain II starts at nucleotide 897. Amino acid sequences which were used for designing oligonucleotide probes are underlined. Positions which differ from the protein sequence of HI-30 purified from urine are enclosed in boxes.

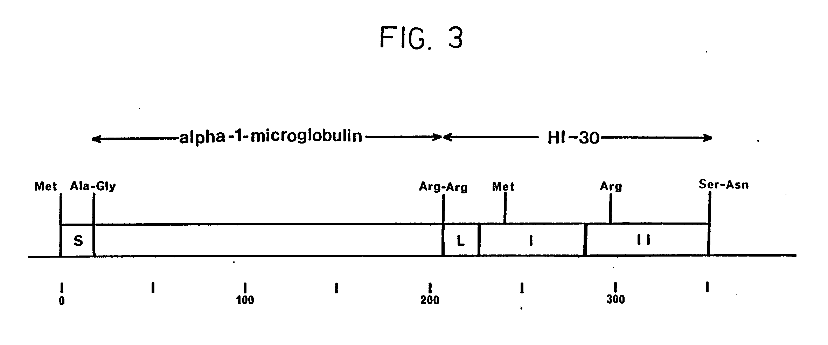

Figure 3 represents the protein structure of the ITI light chain. Met is the first amino acid of the open reading frame. "S" indicates the signal sequence and Ala-Gly represents the potential signal peptidase cleavage site. The Arg-Arg dipeptide is found at the boundary of α-1-microglobulin and HI-30 regions. "L" is the 21 amino acid sequence preceding the Kunitz-like domains I and II. The Ser-Asn dipeptide occurs at the end of the open reading frame.

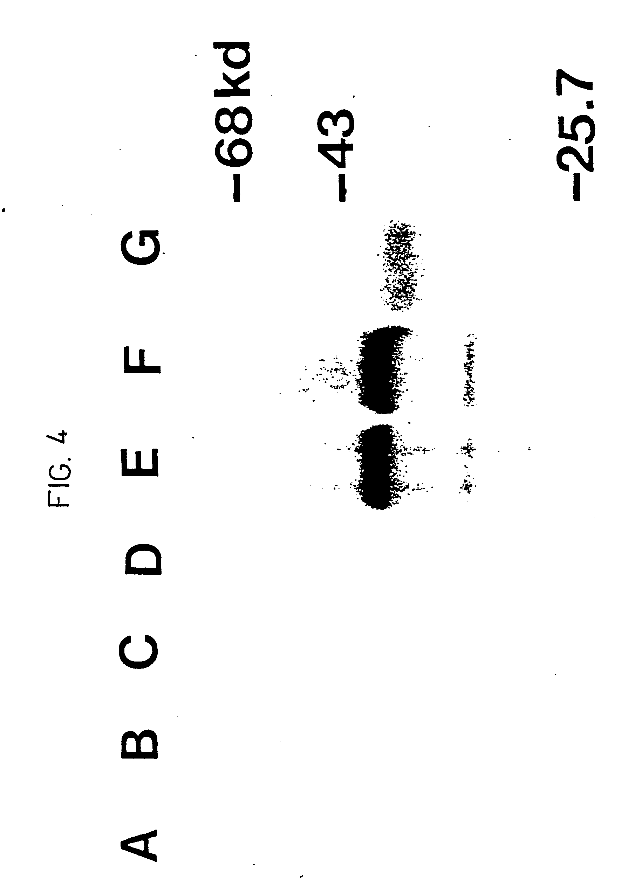

Figure 4 depicts the immunoprecipitation of the translation product of light chain mRNA. RNA was transcribed from the insert cloned into the Bluescribe® expression vector. The RNA was capped in vitro and translated with a micrococcal nuclease-treated rabbit reticulocyte lysate incorporating 35-S methionine. Lane A is a translation with no RNA added and lane G is a translation of light chain mRNA. Lanes B, C, D are light chain RNA translations precipitated with non-immune antibody, anti-β-galactosidase, and no antibody. Lanes E and F were precipitated with the anti-α-1-microglobulin and the anti-inter-α-trypsin inhibitor, respectively.

## Detailed Description of the Invention

The approach to the isolation of this new gene, which was found to encode for both alpha-1-microglobulin and the HI-30 portion of ITI, was to screen a gene library to identify putative clones containing the HI-30 gene by use of specific long oligodeoxyribonucleotide probes. These probes were designed by selecting amino acid regions of HI-30 with low codon degeneracy and converting (reverse translating) this amino acid sequence into a unique DNA sequence using most preferred human codons, that is, codons which are used most frequently in genes coding for human proteins. The protein sequences utilized in this design and the synthetic oligomers prepared are given in Figure 1. One probe represents the amino acid sequence from Met-49 to Phe-66 which is located in the first anti-proteolytic domain of HI-30. Another probe was designed based on amino acid Cys-115 to Cys-132, found in the second domain. Each probe was constructed from two 33 nucleotide long oligomers which overlapped by 12 complementary oligonucleotides at the central region.

## Synthesis of Oligonucleotide Probes

The oligonucleotide probes were prepared using solid-phase synthetic methods (Alkinson et al, 1984). The scheme for synthesis of the oligomers was as outlined by Matteucci et al (1981) utilizing proton activated, protected 2'-deoxy-ribonucleotide phosphoramidites (Beaucage et al, 1981). All sequential steps were performed in an automated manner on an Applied Biosystems Model 380 DNA Synthesizer using protected nucleotides, solvents, chemicals and reagents, all of which were obtained from Applied Biosystems, Foster City, California U.S.A. The solid-phase support (also from Applied Biosystems) was controlled pore glass to which the starting 3'-nucleotide was already attached. Certain modifications were introduced into the automated reaction cycle in accordance with the manufacturer's recommendations. Upon completion of the synthesis, the oligomers were deblocked and cleaved from the solid support within the DNA synthesizer according to the manufacturer's recommendations.

Removal of the blocking groups was completed by heating the aqueous solution containing the oligomer with concentrated ammonium hydroxide at 55° centigrade (C) for from 4 to 24 hours in a sealed vial. The resulting solution was evaporated, the residue dissolved in 0.01 (molar) M triethylammonium bicarbonate buffer, pH 7.0 (TEAB buffer). This solution was chromatographed over Sephadex-G50® Gel Filtration Resin. This column was prepared in, and eluted with, the same TEAB buffer. Material eluting with the void volume was pooled and the solution evaporated. A portion of the residue (10 to 40% of the absorbance units at 260 nanometers), dissolved in loading buffer (composition: 0.1% Bromophenol Blue, 0.1% Xylene Cyanol, 10 millimolar disodium EDTA, in formamide) was further purified by electrophoresis on polyacrylamide gels. The gel size was 18 × 32 centimeters (cm) with a thickness of 1.5 millimeters (mm). The well size for each oligomer purified in this manner was 2 to 5 cm in width and up to five oligomers were purified using a single gel. The concentration of acrylamide in the gel varied from 14 to 20%, depending on the chain length of the desired product. For longer oligomers, the 14% gel is preferred, while shorter oligomers were purified on up to a 20% acrylamide gel. The gel also contained 7M urea and Tris-borate-EDTA buffer (0.1 M Tris, 0.1M borate, 2 millimolar EDTA, pH 8.3). The running buffer was the same Tris-Borate-EDTA mixture. Electrophoresis was carried out at 20 to 60 watts, constant power, for from 6 to 18 hours.

Following completion of the electrophoresis, the gel was encased in plastic wrap and the oligomers were visualized by shadowing with ultraviolet light. This shadowing was accomplished by placing the wrapped gel on a fluorescent thin layer chromatography plate and viewing the gel with a short wave length ultraviolet light source. The desired product appeared as the slowest migrating, major blue band by this shadowing technique. The desired band was excised from the gel. The DNA oligomer was eluted from the gel slice onto powdered diethylaminoethyl (DEAE) cellulose using an EpiGene (Baltimore, Maryland, U.S.A.) D-Gel® electrophoresis apparatus. The oligomer was recovered from the cellulose by elution with 1 M TEAB buffer. The buffer solution containing the oligomer was evaporated, the residue dissolved in 0.01 M TEAB buffer, and then desalted by passage over a column of Sephadex-G50® as described previously. The material eluting in the void volume was pooled and lyophilized to give the final product. Using these procedures, about 0.5 to 5.0 A260 units of each of the purified oligomers was obtained.

The purified oligonucleotide probes were radiolabeled by annealing the individual oligomers and filling-in the remaining single stranded regions with Klenow fragment and radioactive nucleotides by the following methods. The oligomers were first annealed at a concentration of 200 microgram per milliliter ($\mu$g/ml) in 0.1M NaCl. The mixture was heated at 65°C for 10 minutes, 37°C for 20 minutes, 15°C for 20 minutes and 4°C for 20 minutes. The probes were made radioactive by enzymatic filling-in with Klenow fragment from E. coli DNA polymerase I and appropriate radioactive nucleotides. The synthesis reaction consisted of 0.2 $\mu$g annealed oligomers, 400 micromolar ($\mu$M) dTTP, 400 $\mu$M dATP, 100 microcurie ($\mu$Ci) $\alpha$-P32-dCTP (3000 Curie per millimole), 100 $\mu$Ci $\alpha$-P32-dGTP (3000 Curie per millimole), 50 millimolar (mM) Tris (pH 7.2), 10 mM MgCl$_2$, 0.1 mM dithiothreitol, 50 $\mu$g/ml bovine serum albumin, and 10 units Klenow fragment, in a volume of 30 microliter ($\mu$l). The reaction was incubated at 23°C for 60 minutes and then was made 400 $\mu$M with respect to unlabeled dCTP and dGTP. After 15 minutes at 23°C the reaction was stopped with 1 $\mu$l of 10% sodium dodecyl sulfate (SDS) and the labeled oligomers purified by chromatography on Sephadex G-50®. Using this method, specific activities of 10$^9$ cpm/$\mu$g were routinely reached. The two filled-in probes were pooled for initial screening of the library.

Construction of mRNA Library

A lambda gt11 cDNA phage library of human mRNA was constructed as described by Huynh et al., 1985. The library consisted of 10$^6$ independent clones of which 71% of the packaged phage contained inserts. The library was screened as described by Benton et al, 1977.

A total of 10$^6$ independent phage were plated on the host E. coli strain, Y1088, at a density of 375 phage/cm$^2$. The nitrocellulose filters used for plaque lifts were prehybridized for at least 4 hours at 37°C in 20% formamide, 2X Denhardt's solution, 5X SSPE (20X SSPE is 3.6M NaCl, 200 mM NaH$_2$PO$_4$, pH 7.4, 20 mM EDTA, pH 7.4), 0.1% SDS, 100 $\mu$g/ml sheared, single stranded salmon sperm DNA. The filters containing the lifted plaques were then hybridized overnight in the same conditions with 10$^6$ cpm/ml of the radiolabeled oligomer probe mixture. The probe mixture was incubated at 100°C for 10 minutes before adding to the filters. The filters were washed in 0.2X SSPE, 0.1% SDS at 37°C and exposed to Kodak XAR-2 film using Dupont Hi-Plus intensifying screens at -70°C.

By these methods, more than 100 positive clones were identified in the 426,000 phage which were screened. Twelve positive clones were selected and purified by the isolation of single phage plaques. These individual clones were then analyzed by characterizing their melting temperature, i.e., temperature of thermal denaturation of the double stranded probe-DNA complex with each probe and determining the size of the inserted DNA. All twelve clones hybridized to each individual probe. The probe for domain I remained hybridized to each of the clones at 60°C in 0.1X SSPE, 0.1% SDS. The probe for domain II washed off between 50°C and 60°C. The size of the inserts ranged from about 700 to about 1300 nucleotides as determined by polyacrylamide gel electrophoresis of the cloned fragment after removal with EcoRI restriction endonuclease from the identified recombinants. The clone with the largest insert was chosen for further analysis by DNA sequencing.

DNA Sequencing

The DNA fragment inserted in the phage vector was excised from the vector using the restriction enzyme, EcoRI. The released fragment was gel purified and used to generate a random library of smaller fragments in the M13 vector, mp-18, using the sonication technique of Deininger, 1983. Individual M-13 clones were sequenced by the dideoxy chain termination method of Sanger et al, 1977. Twenty random clones were first used to determine the DNA sequence. Four oligodeoxynucleotide primers were then designed and synthesized. These primers were used to confirm remaining regions of ambiguous sequence. Each nucleotide in the gene was confirmed by sequencing the region at least three times and 95% of the nucleotides were sequenced four times.

Data from the shotgun DNA sequencing was aligned using the GEL program of Intelligenetics. An open reading frame search was done with the DNA-Protein Sequence Analysis software purchased from International Biotechnologies, Inc., New Haven, Connecticut, U.S.A. The DNA sequence which was determined is presented in Figure 2. Figure 2 reveals that the EcoRI fragment of this clone is 1,232 nucleotides long. The fragment contains an open reading frame of 352 amino acids which initiates with a methionine codon at nucleotide position 51 and terminates with a stop codon starting at nucleotide 1106. The clone also contains an eight nucleotide stretch of adenylate residues adjacent to the EcoRI site at the 3' end. The signal for poly(A) addition, AATAAAA, is found 19 nucleotides upstream from the poly(A) tail. The oligonucleotides used to construct the probes have high homology with the determined DNA sequence. The domain I probe has 94% homology with the cDNA and the domain II probe has 91% homology. These high levels of homology are consistent with the melting characteristics of these probes.

The complete amino acid sequence of HI-30 can be translated from the nucleotide codons between bases 665 to 1100 at the 3' end of the open reading frame. The amino acid sequence of HI-30 predicted by the DNA sequence is almost identical to that determined for HI-30 purified from human urine. There are minor differences at two locations. The previously published protein sequence assigns Val-Ile at amino acid positions 86-87 and Glu at position 138. The DNA sequence determined herein for the ITI light chain assigns an Ile-Val pair and a Gly at these positions, respectively. The DNA sequence is almost identical to that determined previously by other workers for a cDNA clone from human liver. These workers report only the last 264 nucleotides of a similar mRNA (Bourguinon et al, 1985). The only difference between these two sequences is an extra cytosine in our sequence found at position 1183 in the 3' untranslated region.

The amino terminus of the open reading frame contains a 205 amino acid sequence which has not been previously identified with either 180 kd ITI or HI-30. To determine if this protein sequence might be related to that of other serum proteins, this sequence was used to search the National Biomedical Research Foundation Protein Sequence database. This database was searched with the IFIND program of Intelligenetics. The sequences were aligned after positive matches were identified using the ALIGN program from the same vendor. The sequence showed an almost perfect match with the sequences of two proteins, alpha-1-microglobulin and protein HC. The sequence of protein HC is more complete, however. In relation to protein HC, there is a mismatch at position 55 and a two amino acid gap at positions 56 and 57. The sequence stops three amino acids short of the Arg-Arg dipeptide adjacent to the first amino acid of the HI-30 region. Both alpha-1-microglobulin and protein HC sequences begin at position Gly-20 of the amino terminal domain of the ITI light chain.

Figure 3 summarizes the structure of the ITI light chain. It contains a signal sequence followed by the sequence of alpha-1-microglobulin. At the junction of the alpha-1-microglobulin and HI-30 coding regions are two arginine residues. The sequence of HI-30 begins immediately after these residues and terminates with an additional Ser-Asn dipeptide. The protein has a calculated molecular weight of 42 kd which is similar in size to the ITI light chain synthesized in baboon liver.

## In vitro RNA Transcription

To demonstrate the structure of this newly discovered protein, an mRNA transcript from the cDNA clone of the ITI light chain gene was synthesized as follows. To prepare the specific mRNA, the ITI light chain clone was ligated into the EcoRI site of the Bluescribe® RNA expression vector which was purchased from Stratagene Cloning Systems, Inc. (San Diego, California, U.S.A.) This construct was cut with HindIII and then transcribed in vitro using T7 RNA polymerase according to the method of Krieg et al, 1984. This produced an mRNA copy of the ITI light chain DNA insert. Analysis of the transcription product by gel electrophoresis showed that the mRNA was a full length copy of the DNA. The mRNA transcript was then extracted with phenol and ethanol precipitated. Prior to translation, infra, the RNA transcript was capped with vaccinia virus guanylyltransferase. The reaction mixture contained 50 $\mu$M Tris (pH 7.9), 1.25 $\mu$M MgCl$_2$, 6 mM KCl, 2.5 mM dithiothreitol, 100 $\mu$g/ml bovine serum albumin, 100 $\mu$M S-adenosyl methionine and 330 $\mu$M GTP. One unit of enzyme, which was purchased from Bethesda Research Laboratories, was used per $\mu$g of mRNA. The reaction was carried out at 37°C for 45 minutes. It was terminated by the addition of SDS (to 0.5%) and then phenol extracted.

## In vitro-Translation and Protein Production

Capped mRNAs produced in the above reaction were translated to the corresponding protein in the micrococcal nuclease-treated rabbit reticulocyte lysate translation system purchased from Promega Biotech., Madison, Wisconsin, U.S.A. The translations were performed as described by Pelham et al, 1976.

The protein product thus obtained was then challenged with antibodies to alpha-1-microglobulin and to inter-alpha-trypsin inhibitor. These rabbit antibodies (IgG fraction) were purchased from Accurate Chemical and Scientific Corporation (Westbury, New York, U.S.A.) Immuno-precipitations were performed as described by Dobberstein et al, 1979. The immuno-precipitated products were characterized by electrophoresis on a polyacrylamide gel as described by Laemmli, 1970. Samples were boiled for 10 minutes in 4% SDS and 50 mM dithiothreitol before loading onto the gel. The results of these experiments are shown in Figure 4.

The in vitro translation product is a 42 kd protein (lane G) which is consistent with the size predicted by the open reading frame in the newly cloned gene. The protein band is slightly distorted by co-migrating protein in the lysate. The new protein product is precipitated with antibodies to both alpha-1-microglobulin and 180 kd ITI (lanes E and F). No significant amount of protein product is precipitated by non-immune antibody, anti-beta-galactosidase antibody or when no antibody is added (lanes B, C, and D, respectively). Thus the ITI light chain mRNA encodes for a new protein containing the amino acid sequences of alpha-1-microglobulin with HI-30. Both regions of the fusion protein product are apparently properly folded to present epitopes which are recognized by specific antibodies made to mature 31 kd alpha-1-microglobulin and the high molecular weight form of ITI.

The skilled artisan will appreciate that protein production utilizing the cDNA for the ITI light chain may also be accomplished in vivo. For example, the cDNA of the ITI light chain may be incorporated into a suitable cloning vector (i.e., transfer vector) such as a plasmid or the DNA of certain bacteriophage. Plasmid vectors contain (or may be engineered to contain) various restriction sites suitable for opening the circularized plasmid structure and the subsequent ligation of the DNA sequence into said vector. Bacteriophage DNA may carry an inserted segment of heterologous DNA in place of certain non-essential phage genes. In either instance, the vector provides a means for the introduction of the heterologous sequence into the host, said vector also carrying the genetic information necessary to provide for self-replication therein. The host cell may be selected from among the various procaryotic and eucaryotic hosts now conventional in the art which are used for gene expression. Such organisms include, for example, various strains of E. coli, Bacillus subtilis, Saccharomyces cerevisiae, and the like. Further, the host may be mammalian in nature and include such cell lines as baby hamster kidney cells, Chinese hamster ovary cells, HeLa and VERO cell lines.

Such in vivo protein production may be exemplified as follows. The cDNA sequence encoding the ITI light chain is ligated into the unique EcoRI site of the expression vector pKK223-3 which is commercially available from Pharmacia, Inc. (Molecular Biologicals Catalog, p. 63, 1984). Insertion of the cDNA sequence of the ITI light chain in the correct orientation is then determined by restriction mapping techniques resulting in a recombinant plasmid. Said plasmid is then used to transform E. coli JM105 cells by conventional techniques. The transformed bacteria are then cultured under conditions suitable for growth. The expression of the cDNA encoding the ITI light chain is achieved by induction with isopropyl-β-thiogalactoside. Processing of the microorganism by techniques standard in the art is followed by subsequent isolation of the resultant α-1-microglobulin-HI-30 fusion protein.

As noted previously, ITI is a serine protease inhibitor found in human plasma and serum. This protein has been reported to inhibit the proteolytic enzymes trypsin, chymotrypsin and neutrophil elastase. The most important proteinase inhibitory activity, from a therapeutic standpoint, is the inhibition of the hydrolytic enzyme elastase which may be released from several sources within the human body. ITI can be used therapeutically to treat diseases which result from an excessive release of hydrolytic enzymes, especially elastase from various sources. For example, an excessive release of pancreatic elastase results in pancreatitis. Excessive serum elastase levels have been implicated in atherosclerosis and it is well known that release of excessive neutrophil elastase is observed in acute and chronic inflammation resulting in damage to all connective tissue, blood vessel walls and in necrosis and degeneration of lung tissue. Equally important is the part played by lysosomal enzymes, in particular neutrophil elastase, in inflammatory reactions due to immunological processes, for example, rheumatoid arthritis.

## Bibliography

The following publications which have been referred to in the instant specification are expressly incorporated herein by reference:

1. Alkinson, T. et al in "Oligonucleotide Synthesis - A Practical Approach", Gait, M., Ed.; IRL Press, Oxford England, 1984 (Chapter 3)

2. Beaucage, S. et al, Tetrahedron Letters 22, 1859-1862 (1981)

3. Benton, W. et al, Science 196, 180-182 (1977)

4. Bourguignon, J. et al, FEBS Lett. 162, 379-383 (1983)

5. Bourguignon, J. et al, Biochem. Biophys. Res. Comm. 131, 1146-1153 (1985)

6. Deininger, P. et al, Anal. Biochem. 129, 216-223 (1983)

7. Dobberstein, B. et al, Cell 17, 759-769 (1979)

8. Huynh, T. et al, in DNA Cloning, Glover, D., Ed., IRL Press, Oxford, England (Volume I, 49-78, 1985)

9. Krieg, P. et al, Nucl. Acids Res. 12, 7035-7056 (1984)

10. Laemmli, U., Nature (London) 227, 680-685 (1970)

11. Matteucci, M. et al, J. Am. Chem. Soc. 103, 3185 (1981)

12. Pelham, H. et al, Eur. J. Biochem. 67, 247-256 (1976)

13. Sanger, F. et al, Proc. Natl. Acad. Sci. USA 74, 5463-5467 (1977)

14. Wachter, E. et al, Hoppe-Seyler's Z. Physiol. Chem. 362, 1351-1355 (1981)

## Claims

1. A cDNA sequence encoding alpha-1-microglobulin in sequence with the HI-30 coding region of the inter-alpha-trypsin inhibitor light chain.

2. The cDNA sequence of Claim 1 additionally comprising a DNA sequence coding for a secretory leader sequence 5' to said cDNA sequence.

3. A recombinant DNA cloning vector comprising the cDNA sequence of Claim 1.

4. A recombinant DNA cloning vector comprising the cDNA sequence of Claim 2.

5. A microorganism transformed by the cloning vector of Claim 3.

6. A microorganism transformed by the cloning vector of Claim 4.

7. A fusion protein of the inter-alpha-trypsin light chain comprising α-1-microglobulin and HI-30.

Claims for the following Contracting State : AT

1. A method for isolating a cDNA sequence encoding alpha-1-microglobulin in sequence with the HI-30 coding region of the inter-alpha-trypsin inhibitor light chain comprising the steps of: (a) preparing a cDNA phage library of human mRNA; (b) preparing radiolabeled oligonucleotide probes complementary to said HI-30 coding region; (c) using said probes to screen said library of step (a); and (d) isolating from said library said cDNA sequence encoding alpha-1-microglobulin in sequence with the HI-30 coding region of the inter-alpha-trypsin inhibitor light chain.

32. The method of the claim 1 wherein the cDNA sequence encoding alpha-1-microglobulin in sequence with the HI-30 coding region of the inter-alpha-trypsin inhibitor light chain additionally contains a DNA sequence coding for a secretory leader sequence 5' to said cDNA sequence.

# FIG. 1

DOMAIN I

```
    Met Ala Cys Glu Thr Phe Gln Tyr Gly Gly Cys Met Gly Asn Gly Asn Asn Phe
5'-ATG GCC TGC GAG ACC TTC CAG TAC GGC GGC TGC-3'
                               3'-ATG CCG CCG ACG TAC CCG TTG CCG TTG TTG AAG-5'
```

DOMAIN II

```
    Cys Gln Gly Asn Gly Asn Lys Phe Tyr Ser Glu Lys Glu Cys Arg Glu Tyr Cys
5'-TGC CAG GGC AAC GGC AAC AAG TTC TAC AGC CAG-3'
                           3'-AAG ATG TCG GTC TTC CTC ACG TCT CTC ATG ACG-5'
```

# FIG. 2

```
        10              20              30              40              50              60
         *               *               *               *               *               *
AA TTC TAG ACC GAG CCT GTG GGA TAT ACC AAG GCA GAG GAG CCC ATA GCC ATG AGG AGC CTC
                                                                    Met Arg Ser Leu

        70              80              90             100  α-1-mic              120
         *               *               *               *                        *
GGG GCC CTG CTC TTG CTG CTG AGC GCC TGC CTG GCG GTG AGC GCT|GGC CCT GTG CCA ACG
Gly Ala Leu Leu Leu Leu Leu Ser Ala Cys Leu Ala Val Ser Ala|Gly Pro Val Pro Thr

       130             140             150             160             170             180
        *               *               *               *               *               *
CCG CCC GAC AAC ATC CAA GTG CAG GAA AAC TTC AAT ATC TCT CGG ATC TAT GGG AAG TGG
Pro Pro Asp Asn Ile Gln Val Gln Glu Asn Phe Asn Ile Ser Arg Ile Tyr Gly Lys Trp

       190             200             210             220             230             240
        *               *               *               *               *               *
TAC AAC CTG GCC ATC GGT TCC ACC TGC CCC TGG CTG AAG AAG ATC ATG GAC AGG ATG ACA
Tyr Asn Leu Ala Ile Gly Ser Thr Cys Pro Trp Leu Lys Lys Ile Met Asp Arg Met Thr

       250             260             270             280             290             300
        *               *               *               *               *               *
GTG AGC ACG CTG GTG CTG GGA GAG GGC GCT ACA GAG GCG GAG ATC AGC ATG ACC AGC ACT
Val Ser Thr Leu Val Leu Gly Glu Gly Ala Thr Glu Ala Glu Ile Ser Met Thr Ser Thr

       310             320             330             340             350             360
        *               *               *               *               *               *
CGT TGG CGG AAA GGT GTC TGT GAG GAG ACG TCT GGA GCT TAT GAG AAA ACA GAT ACT GAT
Arg Trp Arg Lys Gly Val Cys Glu Glu Thr Ser Gly Ala Tyr Glu Lys Thr Asp Thr Asp

       370             380             390             400             410             420
        *               *               *               *               *               *
GGG AAG TTT CTC TAT CAC AAA TCC AAA TGG AAC ATA ACC ATG GAG TCC TAT GTG GTC CAC
Gly Lys Phe Leu Tyr His Lys Ser Lys Trp Asn Ile Thr Met Glu Ser Tyr Val Val His

       430             440             450             460             470             480
        *               *               *               *               *               *
ACC AAC TAT GAT GAG TAT GCC ATT TTC CTG ACC AAG AAA TTC AGC CGC CAT CAT GGA CCC
Thr Asn Tyr Asp Glu Tyr Ala Ile Phe Leu Thr Lys Lys Phe Ser Arg His His Gly Pro

       490             500             510             520             530             540
        *               *               *               *               *               *
ACC ATT ACT GCC AAG CTC TAC GGG CGG GCG CCG CAG CTG AGG GAA ACT CTC CTG CAG GAC
Thr Ile Thr Ala Lys Leu Tyr Gly Arg Ala Pro Gln Leu Arg Glu Thr Leu Leu Gln Asp

       550             560             570             580             590             600
        *               *               *               *               *               *
TTC AGA GTG GTT GCC CAG GGT GTG GGC ATC CCT GAG GAC TCC ATC TTC ACC ATG GCT GAC
Phe Arg Val Val Ala Gln Gly Val Gly Ile Pro Glu Asp Ser Ile Phe Thr Met Ala Asp

       610             620             630             640             650             660
        *               *               *               *               *               *
CGA GGT GAA TGT GTC CCT GGG GAG CAG GAA CCA GAG CCC ATC TTA ATC CCG AGA GTC CGG
Arg Gly Glu Cys Val Pro Gly Glu Gln Glu Pro Glu Pro Ile Leu Ile Pro Arg Val Arg
```

# FIG. 2   (continued)

**HI-30**

```
              680         690         700         710         720
               *           *           *           *           *
AGG GCT GTG CTA CCC CAA GAA GAG GAA GGA TCA GGG GGT GGG CAA CTG GTA ACT GAA GTC
Arg Ala Val Leu Pro Gln Glu Glu Glu Gly Ser Gly Gly Gly Gln Leu Val Thr Glu Val

              740         750         760         770         780
               *           *           *           *           *
ACC AAG AAA GAA GAT TCC TGC CAG CTG GGC TAC TCG GCC GGT CCC TGC ATG GGA ATG ACC
Thr Lys Lys Glu Asp Ser Cys Gln Leu Gly Tyr Ser Ala Gly Pro Cys Met Gly Met Thr

    790         800         810         820         830         840
     *           *           *           *           *           *
AGC AGG TAT TTC TAT AAT GGT ACA TCC ATG GCC TGT GAG ACT TTC CAG TAC GGC GGC TGC
Ser Arg Tyr Phe Tyr Asn Gly Thr Ser Met Ala Cys Glu Thr Phe Gln Tyr Gly Gly Cys

    850         860         870         880         890
     *           *           *           *           *
ATG GGC AAC GGT AAC AAC TTC GTC ACA GAA AAG GAG TGT CTG CAG ACC TGC CGA ACT GTG
Met Gly Asn Gly Asn Asn Phe Val Thr Glu Lys Glu Cys Leu Gln Thr Cys Arg Thr Val

    910         920         930         940         950         960
     *           *           *           *           *           *
GCG GCC TGC AAT CTC CCC ATA GTC CGG GGC CCC TGC CGA GCC TTC ATC CAG CTC TGG GCA
Ala Ala Cys Asn Leu Pro Ile Val Arg Gly Pro Cys Arg Ala Phe Ile Gln Leu Trp Ala

    970         980         990        1000        1010        1020
     *           *           *           *           *           *
TTT GAT GCT GTC AAG GGG AAG TGC GTC CTC TTC CCC TAC GGG GGC TGC CAG GGC AAC GGG
Phe Asp Ala Val Lys Gly Lys Cys Val Leu Phe Pro Tyr Gly Gly Cys Gln Gly Asn Gly

   1030        1040        1050        1060        1070        1080
     *           *           *           *           *           *
AAC AAG TTC TAC TCA GAG AAG GAG TGC AGA GAG TAC TGC GGT GTC CCT GGT GAT GGT GAT
Asn Lys Phe Tyr Ser Glu Lys Glu Cys Arg Glu Tyr Cys Gly Val Pro Gly Asp Gly Asp

   1090        1100        1110        1120        1130        1140
     *           *           *           *           *           *
GAG GAG CTG CTG CGC TTC TCC AAC TGA CAA CTG GCC GGT CTG CAA GTC AGA GGA TGG CCA
Glu Glu Leu Leu Arg Phe Ser Asn ---

   1150        1160        1170        1180        1190        1200
     *           *           *           *           *           *
GTG TCT GTC CCG GGG TCC TGT GGC AGG CAG CGC CAA GCA ACC TGG GTC CAA ATA AAA ACT

   1210        1220        1230
     *           *           *
AAA TTG TAA ACT CCT GAA AAA AAA GGA ATT
```

FIG. 3

FIG. 4

A  B  C  D  E  F  G

−68 kd

−43

−25.7